# FASCICULE DE BREVET EUROPEEN

(11) **EP 0 329 558 B1**
(45) Date de publication et mention de la délivrance du brevet: **02.11.1994**
(21) Numéro de dépôt: 89400440.7
(22) Date de dépôt: 17.02.1989
(51) Int. Cl.: C12P 19/04, C12P 19/06

(54) **Procédé de fermentation en deux étapes pour la production de polysaccharides**
Verfahren zur Fermentierungen in zwei Phasen bei der Herstellung von Polysacchariden
Two-stage fermentation process for the production of polysaccharides

(30) Priorité: 18.02.1988 FR 8801933
(43) Date de publication de la demande: 23.08.1989
(73) Titulaire: SANOFI, 75008 Paris (FR)
(72) Inventeur: Eyssautier, Bruno, F-50500 Carentan (FR)
(74) Mandataire: Gillard, Marie-Louise

(56) Documents cités:
- GB-A- 2 090 847
- JOURNAL CHEM. TECH. BIOTECHNOL; 1987, vol. 39, Society of Chemical Industry, GB; L. DE VUYST et al., pp. 263-273#
- DEVELOPMENTS IN INDUSTRIAL MICROBIOLOGY, vol. 21, Proceedings of the 36th General Meeting of the Society for Industrial Microbiology, Pittsburgh, PA (US), 11-17 août 1979, chapitre 1979, Arlington, VA (US); W.L. GRIFFITH et al., pp. 451-460#
- PATENT ABSTRACTS OF JAPAN, vol. 9, no. 188 (C-295)[1911], 03 août 1985#
- JOURNAL OF APPLIED BACTERIOLOGY, vol. 53, 1982, Society for Applied Bacteriology; I.W. SUTHERLAND, pp. 385-393#

## Description

La présente invention concerne un procédé de production de polysaccharides par fermentation, en deux phases, tel que, pendant la première phase de constitution de la biomasse, il n'y ait pratiquement pas de polysaccharide présent dans le milieu de culture.

La plupart des microorganismes producteurs de polysaccharides, bactéries ou champignons, sécrètent le polymère pendant leur croissance, et les milieux de préculture deviennent rapidement visqueux; ceci diminue l'efficacité de l'agitation et de l'aération du milieu et a pour conséquence de limiter la vitesse de formation de la biomasse, et la concentration cellulaire maximale que l'on peut atteindre. Diverses solutions ont été proposées pour dissocier la croissance cellulaire de la production du polysaccharide. On peut citer, dans un procédé continu de production de xanthane, l'utilisation d'un milieu appauvri en hydrates de carbone, comme décrit dans US 3 328 262 ou US 3 251 749 ou encore, comme décrit dans EP-A-0 112 661, l'introduction dans le milieu de préculture, comme seule source de carbone, d'un composé qui ne permette pas la biosynthèse du polysaccharide, tel que le xylose ou le glycérol pour le Xanthomonas campestris, producteur de xanthane.

Mais, dans le premier cas, il est difficile de contrôler la concentration en hydrates de carbone pour n'avoir qu'une faible biosynthèse de polysaccharides tout en maintenant une croissance satisfaisante, tandis que, dans le second cas, il faut noter que des sources de carbone bloquant la sécrétion du polysaccharide au cours de la croissance ne sont connues que pour quelques genres de microorganismes et que surtout elles ne semblent pas utilisables pour toutes les souches de ce microorganisme.

La présente invention ne consiste pas à inhiber la sécrétion du polysaccharide au cours de la phase de préculture mais à le décomposer, par voie enzymatique, au fur et à mesure de sa formation.

Un premier objet de l'invention est un procédé d'obtention d'une masse de microorganismes producteurs de polysaccharide viscosifiant, qui consiste à multiplier ces microorganismes dans un milieu favorable à leur croissance qui contient une enzyme catalysant la décomposition du polysaccharide produit.

Le procédé peut être mis en oeuvre pour l'obtention d'une biomasse de bactéries ou de champignons producteurs de polysaccharide épaississant, tels que les bactéries du genre Xanthomonas productrices de xanthane, et notamment les espèces Xanthomonas begioniae, Xanthomonas campestris, Xanthomonas vesicatoria et Xanthomonas pisi, les bactéries du genre Azobacter productrices d'acide alginique et en particulier celles de l'espèce Azobacter vinelandii, les bactéries de l'espèce Agrobacterium radiobacter et de celles de l'espèce Alcaligenes faecalis et les Pseudomonas myxogenes productrices de succinoglucanes, les bactéries de l'espèce Leuconostoc mesenteroides productrices de dextrane et les champignons de l'espèce Sclerotium producteurs de scléroglucane, tels que ceux décrits dans le brevet US 3 301 848.

Les enzymes mises en oeuvre sont choisies en fonction du polysaccharide produit

Pour l'obtention de Xanthomonas, on peut citer le complexe enzymatique produit par une souche de Bacillus pure ou en mélange avec une souche de Flavobacterium décrites dans Dev. Ind. Microbiol. 26 p. 281-288 (1984) ou le complexe décrit dans J. Ind. Microbiol. p. 31-37 (1986).

Pour la production de Sclerotium, on peut citer la bêta-1,3 glucanase de Basidiomycetes mentionnée par F.E. Halleck dans US 3 423 288 ou celle obtenue par culture de Sporotrichum dimorphosporum, notamment de la souche déposée à l'ATCC sous le n° 24562 et la bêta-1,3 bêta-1,6 glucanase commercialisée par Novo (Danemark) sous la marque Glucanex ou encore celle commercialisée par Gist Brocades sous la marque Rapidase GL 150.

Pour l'obtention de Leuconostoc mesenteroides, on peut citer la dextranase de Streptococcus mutans décrite dans "Molecular Microbiology and Immunology of Streptococcus mutans" S. Humada et al.-Elsevier Sc. Pub. (1986) p. 205-215 ou la dextranase fongique, issue de Penicillium lilacinum commercialisée par Novo ainsi que les bêta-1,3 bêta-1,6 glucanases.

Les quantités d'enzyme à introduire dans le milieu de préculture sont fonction de la nature et de la pureté de l'enzyme, du polysaccharide et de sa vitesse de production en phase de croissance; des essais préalables permettront au spécialiste de déterminer dans chaque cas la concentration efficace.

De même, on choisira parmi les enzymes disponibles dégradant le polysaccharide considéré celles qui ont une bonne activité enzymatique aux conditions de pH et de température de la préculture et qui peuvent être inactivées avant ou pendant la phase de production.

En effet, un autre objet de l'invention est le procédé de production d'un polysaccharide par fermentation, en deux étapes, qui consiste à préparer une certaine quantité de microorganismes sécréteurs par une préculture en présence d'une enzyme hydrolysant le polysaccharide puis à faire produire le polysaccharide par les microorganismes dans un milieu adapté à la production. Un tel procédé a notamment pour avantage de donner sensiblement la même quantité de polysaccharide qu'avec un procédé classique, mais dans des temps nettement plus courts.

Les conditions de préculture et de fermentation peuvent être éventuellement adaptées pour favoriser l'hydrolyse pendant la croissance et au contraire l'inhiber pendant la phase de production; le choix notamment du pH des milieux de préculture et de culture et/ou de la température peut être un moyen de stimuler ou d'inactiver l'enzyme; dans certains cas, les milieux classiques pourront être utilisés. On peut aussi introduire dans le milieu en fin de préculture un répresseur de l'enzyme ou un effecteur allostérique.

On peut aussi, pour neutraliser l'activité enzymatique, en fin de préculture, porter transitoirement l'inoculum à une température, ou un pH, qui dénature l'enzyme choisie.

Dans la phase de préculture, le produit servant de source de carbone au microorganisme peut être introduit dans le milieu de culture en quantité nettement plus faible, si des hydrates de carbone assimilables par les microorganismes sont libérés dans le milieu par l'hydrolyse du polysaccharide. Par exemple, dans une préculture de Sclerotium rolfsii, les teneurs en glucose exogène seront réduites d'au moins 70%.

Une faible activité enzymatique résiduelle dans le milieu, en phase de production, est acceptable dans la mesure où l'hydrolyse du polysaccharide libère des sucres qui peuvent être utilisés comme source de carbone par les microorganismes.

Dans ce qui suit, on décrit à titre d'exemple l'application du procédé selon l'invention à la production de scléroglucane par fermentation du champignon Sclerotium rolfsii.

### EXEMPLE

### Préculture : obtention de Sclerotium rolfsii à partir de sclérotes congelées de la souche ATCC n° 15206

### a) revitalisation du champignon :

On introduit dans un flacon Erlenmeyer contenant 100 ml d'un milieu stérilisé aqueux constitué de 4,5 g de glucose, 0,2 g de NaNO₃, 0,1 g d'extrait de levure, 0,1 g de KH₂PO₄, 0,025 g de MgSO₄ et 0,0005 g de thiamine, 5 sclérotes congelées.

Le flacon fermé est mis à incuber, sous agitation, à 28°C pendant 96 heures.

### b) Préparation d'un premier inoculum dans un fermenteur de 6 litres, agité :

Le contenu de 4 flacons de revitalisation est introduit dans 4 litres d'un milieu stérilisé aqueux constitué de 176 g de glucose, 8 g de NaNO₃, 40 g d'extrait de levure, 4 g de KH₂PO₄, 4 g de MgSO₄, 0,02 g de thiamine et 4 g d'antimousse tel que le Pluronic^{R} puis on ajoute 160 mg de Glucanex ^{R} stérile.

Le milieu est maintenu 44 heures à 28°C sous agitation, le pH étant maintenu à 4,5 par addition d'une solution aqueuse de soude à 10%.

### c) Préparation d'un second inoculum dans un fermenteur de 15 litres, agité :

1 litre de l'inoculum précédemment obtenu est introduit dans 10 litres d'un milieu stérilisé aqueux constitué de 350 g de glucose, 28 g de NaNO₃, 7,2 g d'eau de trempage du maïs (CSL), 7g de KH₂PO₄, 10,5 g de MgSO₄, 0,05 g de thiamine et 5 g de Pluronic et 400 mg de glucanex.

L'ensemble est maintenu 24 heures à 28°C sous agitation.

On obtient ainsi un milieu contenant une quantité importante de biomasse, puisqu'elle est en fin d'incubation de 10 g par kilo de milieu au lieu de 2 g/kg dans une fermentation classique.

### d) Production de scléroglucane:

On introduit dans un fermenteur de 15 litres du même type que celui utilisé pour la préparation de l'inoculum 10 l d'un milieu stérilisé aqueux constitué de 350 g de glucose, 14 g de NaNO₃, 3,6 g d'eau de trempage du maïs (CSL), 10,5 g de MgSO₄, 7 g de KH₂PO₄, 5 g de Pluronic et 1 litre d'inoculum

L'ensemble est incubé à 28°C sous agitation pendant 36 heures 30 minutes, en maintenant le pH à 3 par addition d'une solution aqueuse de NaOH à 10%.

On traite ensuite le milieu de fermentation de façon classique, selon l'application envisagée, soit on sépare la biomasse par filtration et on précipite le scléroglucane par addition d'un solvant alcoolique tel que l'isopropanol, soit on introduit dans le milieu de fermentation un solvant alcoolique et on isole le précipité formé.

On obtient par le procédé de l'invention la même quantité de scléroglucane que dans une fermentation classique sans enzyme, la quantité de polysaccharide formé dépendant essentiellement de la quantité de glucose présente dans le milieu; par contre, la durée de la fermentation dans un procédé classique, sans l'enzyme, serait de 48 heures au lieu de 36 heures 30 minutes.

## Revendications

1. Procédé d'obtention d'une masse de microorganismes producteurs de polysaccharide, caractérisé en ce que la croissance des microorganismes a lieu dans un milieu contenant une enzyme hydrolysant le polysaccharide.

2. Procédé selon la revendication 1, caractérisé en ce que le microorganisme est un champignon du genre Sclerotium, producteur de scléroglucane.

3. Procédé selon la revendication 2, caractérisé en ce que l'enzyme est une bêta-1,3 glucanase.

4. Procédé selon la revendication 2, caractérisé en ce que l'enzyme est une bêta-1,3 bêta-1,6 glucanase.

5. Procédé de production de polysaccharide par fermentation, caractérisé en ce que la phase de croissance des microorganismes a lieu en présence d'une enzyme hydrolysant le polysaccharide.

6. Procédé selon la revendication 5, caractérisé en ce que l'enzyme est inhibée à la fin de la phase de croissance par modification du pH ou de la température.

7. Procédé selon la revendication 6, caractérisé en ce que l'enzyme est inactivée pendant la phase de production par fixation du pH à une valeur convenable.

8. Procédé selon l'une des revendications 5 à 7, caractérisé en ce que le microorganisme est un champignon du genre Sclerotium, producteur de scléroglucane.

9. Procédé selon la revendication 8, caractérisé en ce que l'enzyme est une bêta-1,3 glucanase.

10. Procédé selon la revendication 9, caractérisé en ce que l'enzyme est une bêta-1,3 bêta-1,6 glucanase.

11. Procédé selon l'une des revendications 9 et 10, caractérisé en ce que le pH du milieu de production est 3.

## Claims

1. A process for obtaining a mass of microorganisms producing polysaccharide, wherein the growth of the microorganism takes place in a medium containing an enzyme which hydrolyzes the polysaccharide.

2. A process as claimed in claim 1, wherein the microorganism is a Sclerotium type fungus, producing scleroglucane.

3. A process as claimed in claim 2, wherein the enzyme is a beta-1,3 glucanase.

4. A process as claimed in claim 2, wherein the enzyme is a beta-1,3 beta-1,6 glucanase.

5. A process for producing polysaccharide by fermentation, wherein the microorganisms growth stage takes place in the presence of an enzyme which hydrolyzes the polysaccharide.

6. A process as claimed in claim 5, wherein the enzyme is inhibited at the end of the growth stage by modification of the pH or of the temperature.

7. A process as claimed in claim 6, wherein the enzyme is inactivated during the production stage by fixing the pH at a suitable value.

8. A process as claimed in any one of claims 5 to 7, wherein the microorganism is a scleroglucane-producing Sclerotium type fungus.

9. A process as claimed in claim 8, wherein the enzyme is a beta-1,3 glucanase.

10. Process as claimed in claim 9, wherein the enzyme is a beta-1,3 beta-1,6 glucanase.

11. Process as claimed in any one of claims 9 and 10, wherein the pH of the production medium is 3.

## Patentansprüche

1. Verfahren zur Gewinnung einer Masse von Mikroorganismen als Erzeuger von Polysaccharid, dadurch gekennzeichnet, daß das Wachstum der Mikroorganismen in einem Medium stattfindet, welches ein das Polysaccharid hydrolysierendes Enzym enthält.

2. Verfahren nach Anspruch 1, dadurch gekennzeichnet, daß der Mikroorganismus ein Pilz der Gattung Sclerotium ist und Scleroglucan erzeugt.

3. Verfahren nach Anspruch 2, dadurch gekennzeichnet, daß das Enzym beta-1,3-Glucanase ist.

4. Verfahren nach Anspruch 2, dadurch gekennzeichnet, daß das Enzym beta-1,3-beta-1,6-Glucanase ist.

5. Verfahren zur Herstellung von Polysaccharid durch Fermentation, dadurch gekennzeichnet, daß die Wachstumsphase der Mikoorganismen in Gegenwart eines das Polysaccharid hydrolysierenden Enzyms stattfindet.

6. Verfahren nach Anspruch 5, dadurch gekennzeichnet, daß das Enzym am Ende der Wachstumsphase durch Veränderung des pH-Werts oder der Temperatur inhihiert wird.

7. Verfahren nach Anspruch 6, dadurch gekennzeichnet, daß das Enzym während der Herstellungsphase durch Fixierung des pH-Werts auf einem geeigneten Wert inaktiviert wird.

8. Verfahren nach einem der Ansprüche 5 bis 7, dadurch gekennzeichnet, daß der Mikroorganismus ein Pilz der Gattung Sclerotium ist und Scleroglucan erzeugt.

9. Verfahren nach Anspruch 8, dadurch gekennzeichnet, daß das Enzym beta-1,3-Glucanase ist.

10. Verfahren nach Anspruch 9, dadurch gekennzeichnet, daß das Enzym beta-1,3-beta-1,6-Glucanase ist.

11. Verfahren nach einem der Ansprüche 9 und 10, dadurch gekennzeichnet, daß der pH-Wert des Herstellungsmediums 3 ist.
